# EUROPEAN PATENT APPLICATION

(11) **EP 4 752 137 A1**
(43) Date of publication of application: **03.06.2026**
(21) Application number: 24786514.0
(22) Date of filing: 24.07.2024
(51) Int. Cl.: C07D 311/30, A61P 35/00, A61K 31/352

(54) **NEW COMPOUNDS DERIVED FROM MORIN, METHOD FOR OBTAINING THEM AND THEIR USES**

(30) Priority: 24.07.2023 ES 202330632
(71) Applicant: Universidad de Sevilla, 41013 Sevilla (ES)
(72) Inventor: TALERO BARRIENTOS, Elena, 41013 SEVILLA (ES); ÁVILA ROMÁN, Francisco Javier, 41013 SEVILLA (ES); GARCÍA GIL, Sara, 41013 SEVILLA (ES); MOTILVA SÁNCHEZ, Virginia, 41013 SEVILLA (ES); RODRÍGUEZ LUNA, Azahara, 41013 SEVILLA (ES); GÓMEZ HURTADO, Mario Armando, 41013 SEVILLA (ES); RODRÍGUEZ GARCÍA, Gabriela, 41013 SEVILLA (ES); DEL RÍO TORRES, Rosa Elva Norma, 41013 SEVILLA (ES)
(74) Representative: Herrero & Asociados, S.L.
(86) International application number: PCT/ES2024/070476
(87) International publication number: WO 2025/022038

(57) **Abstract**

The present invention relates to new compounds derived from morin, to the synthesis and use of the new compounds, with antioxidant and anti-inflammatory activity against ultraviolet (UV) radiation.

## Description

The present invention relates to new compounds derived from morin, to the synthesis and use of the new compounds, with antioxidant and anti-inflammatory activity against ultraviolet (UV) radiation.

### Background of the invention

The skin acts as the first line of defense against various external agents, including ultraviolet (UV) radiation, which causes oxidative damage to skin, accompanied by an inflammatory response and dysfunction of the skin barrier. Exposure to UV radiation is known to be the main risk factor for the development of skin cancer, which accounts for one in three cases of cancer worldwide, according to the WHO. Despite being one of the most easily preventable types of cancer, the number of cases is increasing. Furthermore, UVB radiation is closely related to skin ageing, which is clinically characterized by dryness, pigmentation, laxity, wrinkles and erythema. Tissue damage is reflected in functional and structural alterations, with an inflammatory basis, which affects components of the extracellular matrix and causes the activation of proteolytic enzymes, such as metalloproteinases. For this reason, it is essential to use sunscreens that protect the skin from the harmful effects of solar radiation. Currently, the cosmetics industry is making progress in developing new filters and there are increasing awareness campaigns to prevent damage caused by the sun. However, there are factors such as incorrect and insufficient application of sunscreens, lack of reapplication or lack of photostability of the filters that do not ensure complete sun protection, especially in patients with previous dermatological pathologies or who are highly sensitive to solar radiation. For this reason, search for new compounds with antioxidant and anti-inflammatory activity, which can be incorporated as adjuvants in sunscreens and contribute to optimal photoprotection, is of great interest.

The study of natural ingredients is at the forefront of research into new bioactive compounds. Many of these ingredients have been used as biological filters, which are considered substances with antioxidant activity that decrease the production of free radicals, reducing cell damage, and therefore, help to prevent photoaging and skin cancer. Some examples of these products that have demonstrated photoprotective activity include an extract from the fern *Polypodium leucotomos,* an extract from the grass *Deschampsia antarctica,* some mycosporine-type amino acids isolated from algae, carotenoids such as β-carotene or a multitude of phenolic compounds isolated from plants such as *Vitis vinifera L., Ruta graveolens L., or Ginkgo biloba L.,* among others.

Among the wide variety of bioactive natural ingredients, phenolic compounds and carotenoids represent two of the most studied groups, with a wide variety of properties, including antioxidant, anti-inflammatory and antitumor effects, which make them potentially useful as photoprotective agents. Regarding phenolic compounds, those that have the greatest potential to protect the human body against reactive oxygen species (ROS) are a subclass of flavonoids known as flavonols, among which morin, 2-(2,4-dihydroxyphenyl)-3,5,7-trihydroxy-4H-chromen-4-one, or 2',3,4',5,7-pentahydroxyflavone, stand out.

Morin is a polyphenol, originally isolated from members of the Moraceae family, although it is also found in species of the families Rosaceae, Fagaceae and Myrtaceae. This flavonoid has been shown to be an oral chemopreventive agent against carcinogenesis both in vitro and in vivo. Morin has also been evaluated as a component of sunscreens, after its encapsulation in polymeric nanoparticles.

Regarding these applications, morin has been described as part of the composition of nutraceuticals aimed at the prevention/treatment of cancer in patent application US20100209388A1. Additionally, morin has been included in the preparation of mixtures of active ingredients free of metal salts, useful for the treatment of cancer, non-malignant tumors, as well as antibacterial, antifungal and antiviral agents as described in application WO1988003805A1.

As with other naturally occurring polyphenols, morin is a compound that can undergo structural modifications to improve its stability and optimize its applicability. One way to improve the chemical stability and pharmacological capacity of this type of chemical substances with pharmacological potential is chemical derivatization.

Based on this background, there is a great need to find new ingredients that may provide improved antioxidant and anti-inflammatory biological activity compared to already known compounds.

### Description of the invention

In the present invention, the carbonyl group of morin, also known as morin or 2-(2,4-dihydroxyphenyl)-3,5,7-trihydroxy-4H-chromen-4-one, is used to prepare Schiff bases, which are known as azomethines or imines. Structurally, a Schiff base is a nitrogen analogue derived from an aldehyde or ketone in which its carbonyl bond (C=O) is replaced by a double bond (C=N). Therefore, the distinctive functional group of this class of derivatives is represented by the general formula R^{a}R^{b}C=N-R^{c}. The most common method for the preparation of Schiff bases involves condensation reactions between the carbonyl of aldehydes or ketones with compounds that have an -NH₂ group, such as primary amines (RNH₂), hydroxylamines (NH₂OH), hydrazines (NH₂NH₂) and semicarbazides (NH₂NHCONH₂), and in which the elimination of a water molecule occurs.

A first aspect of the invention consists of compounds derived from morin of formula (I) wherein the (C=O) group is replaced by a double bond (C=N-R).

Therefore, the first aspect of the invention relates to a compound of formula (I), 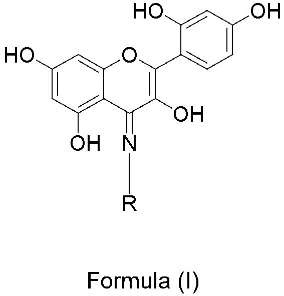
wherein R is selected from: a saturated, straight or branched C₁-C₆ alkyl radical, a straight or branched C₂-C₆ alkenyl radical; a straight or branched C₂-C₆ alkynyl radical, a C₃-C₁₂ cycloalkyl radical, a C₃-C₁₂ aryl radical, a -XR¹ radical, a --NHC=XNR²R³ radical, a -NHR⁴ radical, a -NHCOOR⁵ radical, a -NHCOR⁶ radical;
wherein X is selected from O, S;
wherein R¹, R², R³, R⁴, R⁵, and R⁶ may be the same or different and are selected from H, a saturated, straight or branched C₁-C₆ alkyl radical, a straight or branched C₁-C₆ alkenyl radical; a straight or branched C₁-C₆ alkynyl radical, a C₃-C₁₂ cycloalkyl radical, a C₃-C₁₂ aryl radical.

The method for obtaining the products of formula (I) of the invention is a simple method, with a single reaction step, and with simple reaction conditions that can be carried out in any chemical synthesis laboratory.

The present invention contemplates the preparation of compounds of general formula (I) using morin (2-(2,4-dihydroxyphenyl)-3,5,7-trihydroxy-4H-chromen-4-one), a compound of formula (II) and nitrogen reagents for the formation of azomethine derivatives or Schiff bases in the synthesis steps, in suitable proportions and reaction medium.

Therefore, a second aspect of the invention relates to a method for obtaining compounds of general formula (I) as defined above. 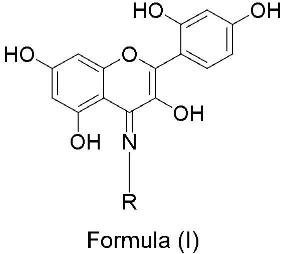 which comprises the steps of:
reacting 2-(2,4-dihydroxyphenyl)-3,5,7-trihydroxy-4H-chromen-4-one of formula (II) 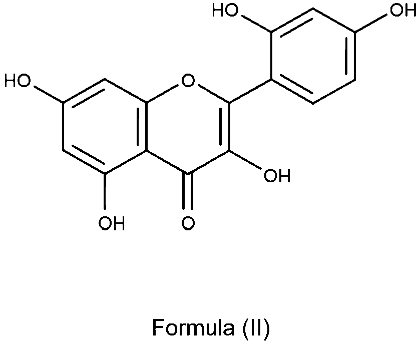
with a nitrogen compound of formula H₂NR, in the presence of an organic solvent;
wherein R is selected from: a saturated, straight or branched C₂-C₆ alkyl radical, a straight or branched C₂-C₆ alkenyl radical; a straight or branched C₁-C₆ alkynyl radical, a C₃-C₁₂ cycloalkyl radical, a C₃-C₁₂ aryl radical, a -XR¹ radical, a -NHC=XNR²R³ radical, a -NHR⁴ radical, a -NHCOOR⁵ radical, a -NHCOR⁶ radical;
wherein X is selected from O, S;
wherein R¹, R², R³, R⁴, R⁵, and R⁶ may be the same or different and are selected from H, a saturated, straight or branched C₁-C₆ alkyl radical, a straight or branched C₁-C₆ alkenyl radical; a straight or branched C₁-C₆ alkynyl radical, a C₃-C₁₂ cycloalkyl radical, a C₃-C₁₂ aryl radical.

These modifications of morin improve the absorption capacity in the UV-visible (vis) range, the stability of these molecules of formula (I), and also maintain their antioxidant and photoprotective activity. For all these reasons, the compounds of formula (I) of the invention can be used as adjuvants in photoprotection, with the aim of preventing the appearance of cancerous lesions, the exacerbation of photodermatoses and, in addition, reducing premature aging of the skin caused by exposure to solar radiation.

The present invention relates to new compounds derived from morin, to the synthesis and use of the new compounds, Schiff bases, with antioxidant and anti-inflammatory activity against UV radiation. Therefore, the applicability of the present invention could be encompassed within the cosmetic and/or dermatological field and, specifically, forming part of photoprotective formulations as an adjuvant for the prevention of skin cancer and cell damage or premature photo-aging of the skin. The compounds of formula (I) can be adjuvants of the photoprotective activity of physical and chemical filters. Then one aspect of the invention relates to the compounds of formula (I) according to the invention for their use as adjuvants of photoprotectors.

These modifications of morin provide a greater capacity for absorbing UV-vis light, improving and/or preserving the photoprotective activity of morin itself.

Therefore, other aspects of the invention are: the use of the product of the invention defined above in the preparation of a cosmetic or dermatological composition to protect a mammal from UV-vis radiation; also an aspect of the invention is a compound of formula (I) for use in the protection of a mammal from UV-vis radiation and finally another aspect of the invention is a compound of formula (I) for use in the prevention of skin cancer and for use in the prevention of skin aging.

Another aspect of the invention is the cosmetic composition or the dermatological composition comprising the product of formula (I) of the invention.

### Brief description of the drawings

Figure 1 shows a comparison of the UV-vis absorption spectra in the 200-500 nm range of morin (solid line), morin oxime (dash-dotted line), and morin semicarbazone (dashed line). The figure illustrates the UV light absorption capacity by plotting the molar extinction coefficient (ε) and wavelength for each of the compounds synthesized in Example 1.
Figures 2A, 2B and 2C show the evaluation of the anti-inflammatory activity as IL-6 production (pg/ml) of morin (2A) and the two compounds obtained in Example 1, morin oxime (2B) and morin semicarbazone (2C) in HaCaT keratinocytes exposed to UVB radiation.
Figures 3A and 3B show the evaluation of the intracellular antioxidant activity (%ROS) of morin (FIG. 3A) and morin semicarbazone (FIG. 3B) obtained in Example 1 in HaCaT keratinocytes exposed to UVB.
Figures 4A, 4B and 4C show the effect of fucoxanthin (4A) and fucoxanthin combined with morin (4B) or morin semicarbazone (4C) from Example 1 on cell viability (%cell viability) in HaCaT keratinocytes exposed to UVB radiation.
Figure 5A, 5B and 5C show the evaluation of intracellular antioxidant activity (%ROS) of fucoxanthin (5A) and fucoxanthin combined with morin (5B) or morin semicarbazone (5C) from Example 1 in HaCaT keratinocytes exposed to UVB radiation.
Figure 6A, 6B and 6C show the evaluation of anti-inflammatory activity (IL-6(pg/ml)) of fucoxanthin (6A) and fucoxanthin combined with morin (6B) or morin semicarbazone (6C) from Example 1 on HaCaT keratinocytes exposed to UVB radiation.

### Description of a preferred embodiment

As stated above, the first aspect of the invention relates to:
A compound of formula (I) 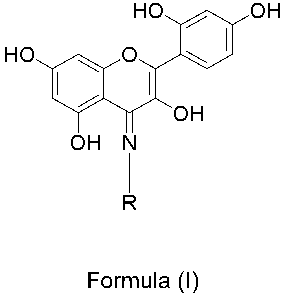
wherein R is selected from: a saturated, straight or branched C₁-C₆ alkyl radical, a straight or branched C₂-C₆ alkenyl radical; a straight or branched C₂-C₆ alkynyl radical, a C₃-C₆ cycloalkyl radical, a C₃-C₆ aryl radical, a -XR¹ radical, a -NHC=XNR²R³ radical, a -NHR⁴ radical, a -NHCOOR⁵ radical, a -NHCOR⁶ radical;
wherein X is selected from O, S;
wherein R¹, R², R³, R⁴, R⁵, and R⁶ may be the same or different and are selected from H, a saturated, straight or branched C₁-C₆ alkyl radical, a straight or branched C₂-C₆ alkenyl radical; a straight or branched C₂-C₆ alkynyl radical, a C₃-C₆ cycloalkyl radical, a C₃-C₆ aryl radical.

As stated above, the term " C₁-C₆ alkyl" refers to a straight or branched hydrocarbon chain radical consisting of 1 to 6 carbons, containing no unsaturation, and which is attached to the moiety of the molecule by a single bond, e.g., methyl, ethyl, 1-propyl, 2-propyl, 1-butyl, t-butyl, 1-pentyl.

In the present invention the term "C₂-C₆ alkenyl" refers to a straight or branched chain of two to six carbon atoms containing one or more double bonds, e.g., ethenyl, 2-propenyl, and the like.

In the present invention the term "C₂-C₆ alkynyl" refers to a straight or branched chain of two to six carbon atoms containing one or more triple bonds, for example, ethynyl, 2-propynyl, and the like.

In the present invention the term "C₃-C₆ cycloalkyl" refers to a straight chain of two to six carbon atoms forming a cyclic structure, such as cyclopropyl.

In the present invention the term "C₃-C₆ aryl" refers to a cyclic aromatic radical such as phenyl.

In the present specification and claims, the term "alkoxy" is used to designate a group of formula: R¹O- wherein R¹ represents that being defined above. Preferably, the term "alkoxy" is used to designate an alkoxy with an alkyl group of 1 to 3 carbon atoms, such as methoxy or ethoxy.

Preferably R is selected from: a saturated, straight or branched C₁-C₃ alkyl radical, a straight or branched C₂-C₃ alkenyl radical; a straight or branched C₂-C₃ alkynyl radical, a -XR¹ radical, a -NHC=XNR²R³ radical, a -NHR⁴, radical, a -NHCOOR⁵ radical, a - NHCOR⁶ radical; wherein X is selected from O, S; wherein R¹, R², R³, R⁴, R⁵, and R⁶ may be the same or different and are selected from H, a saturated, straight or branched C₁-C₃ alkyl radical, a straight or branched C₂-C₃ alkenyl radical; a straight or branched C₂-C₃ alkynyl radical. More preferably R is selected from OR¹ and a - NHC=XNR²R³ radical.

**In** a preferred embodiment, the product of formula (I) is morin oxime, (2-(2,4-dihydroxyphenyl)-3,5,7-trihydroxy-4H-chromen-4-one oxime). In another preferred embodiment, the product of formula (I) is morin semicarbazone, (2-(2-(2,4-dihydroxyphenyl)-3,5,7-trihydroxy-4H-chromen-4-ylidene)hydrazine-1-carboxamide).

A second aspect is the method for obtaining the compounds of formula (I) as defined above. **In** a preferred embodiment, 2-(2,4-Dihydroxyphenyl)-3,5,7-trihydroxy-4H-chromen-4-one of formula (II) is reacted with hydroxylamine hydrochloride or semicarbazide hydrochloride.

Preferably in the method of the invention the organic solvent is selected from: methanol, ethanol, propanol, isopropanol, butanol, iso-, sec- or tert-butanol, pentanol, hexanol, heptanol, octanol or ethylhexanol. More preferably methanol.

The preparation of these compounds of general formula (I) using morin and nitrogen reagents for the formation of azomethine derivatives or Schiff bases can be carried out preferably using equimolar proportions, more preferably with an excess of the nitrogen reagent.

The compound (2-(2-(2,4-dihydroxyphenyl)-3,5,7-trihydroxy-4H-chromen-4-ylidene)hydrazine-1-carboxamide) has demonstrated antioxidant and anti-inflammatory activities, which are enhanced in the presence of fucoxanthin.

Therefore, one embodiment of the invention relates to a composition comprising the compound (2-(2-(2,4-dihydroxyphenyl)-3,5,7-trihydroxy-4H-chromen-4-ylidene)hydrazine-1-carboxamide) and fucoxanthin.

These modifications of morin improve the absorption capacity in the UV-vis range, the stability of these molecules of formula (I), and also maintain their antioxidant and photoprotective activity. For all these reasons, the compounds of formula (I) of the invention can be used as adjuvants in photoprotection, with the aim of preventing the appearance of cancerous lesions, the exacerbation of photodermatoses, and, in addition, reducing premature aging of the skin caused by exposure to solar radiation.

As stated above, the compound of formula (I) of the invention is useful as an adjuvant for photoprotectors. Preferably the photoprotector is fucoxanthin.

### EXAMPLES

The following examples are merely illustrative of this invention and should not be interpreted in a limiting sense thereof.

### Example 1. Synthesis of the compounds morin oxime, (2-(2,4-dihydroxyphenyl)-3,5,7-trihydroxy-4H-chromen-4-one oxime) and morin semicarbazone (2-(2-(2,4-dihydroxyphenyl)-3,5,7-trihydroxy-4H-chromen-4-ylidene)hydrazine-1-carboxamide)

For the preparation of morin oxime, 0.03 g of morin dissolved in 5 mL of MeOH were used, 0.0206 g of hydroxylamine hydrochloride and 0.0206 g of sodium acetate were added. The mixture was left under constant stirring and reflux for 2.5 hours. After this time, the crude product of the reaction was tempered and poured onto wet ice generating a precipitate, which was washed with dichloromethane and methanol obtaining a yellow solid in 60% yield. Yellow crystals were obtained, with a melting point of 290-293 °C and which are soluble in AcOEt, acetone, MeOH, and partially soluble in water. ¹H NMR δ 7.47 d (J = 8.5 Hz). 6.45 dd (J = 8.5; 2.1 Hz). 6.39 d (J = 2.1 Hz). 6.34 d (J = 1.9 Hz). 6.16 d (J = 1.9 Hz). NMR of ¹³C δ 102.9. 122.0. 122.0. 144.3. 149.1. 149.4. 152.8. 153.1. 154.7. 155.9. 84.5. 89.5. 95.5. 95.5. 99.5. UV (EtOH) λₘₐₓ (log *ε*) 210 (4.6). 265 (4.2). 390 (4.4). The preparation of the compound can be carried out using the above-mentioned method with 0.1 g of morin, 0.07 g of hydroxylamine hydrochloride and 0.07 g of sodium acetate.

For the preparation of morin semicarbazone, 0.03 g of morin dissolved in 5 mL of MeOH was used, 0.0332 g of semicarbazide hydrochloride and 0.0332 g of sodium acetate were added. The mixture was left under constant stirring and reflux for 3 hours. After this time, the crude product of the reaction was tempered and poured onto wet ice generating a precipitate, which was recovered by filtration obtaining a coffee solid in 76% yield. Brown crystals were obtained, with a melting point of 267-270 °C, solubility in AcOEt, acetone, MeOH. ¹H NMR (400 MHz, DMSO-d6) 12.63 s. 10.71 sa. 9.77 sa. 7.22 d (J= 8.4 Hz). 6.37 d (J= 2.4 Hz). 6.33 dd (J= 8.4; 2.4 Hz). 6.28 d (J= 1.9 Hz). 6.16 d (J= 1.9 Hz). UV (EtOH) λₘₐₓ (log *ε*) 210 (4.5). 265 (5.2). 390 (5.1). The preparation of the compound can be carried out using the above-mentioned method with 0.1 g of morin, 0.09 g of semicarbazide hydrochloride and 0.09 g of sodium acetate.

### Example 2. Comparison of absorption spectra between morin and the compounds obtained in Example 1

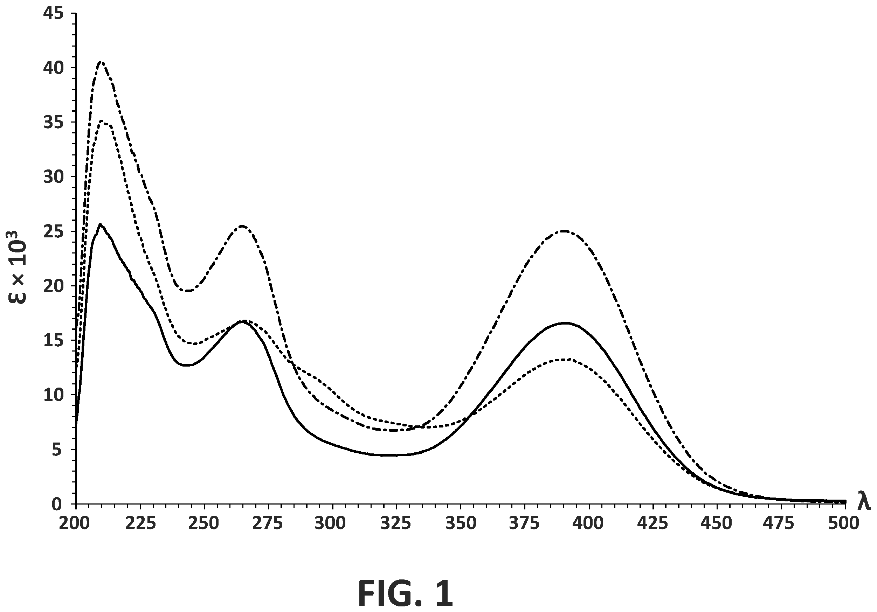

As experimental evidence of the improvement in the photoprotective capacity of compounds synthesized in Example 1, a comparison was made of the absorption capacity in the UV-vis range between morin and nitrogen derivatives (Schiff bases). As can be seen in Figure 1, the UV-vis absorption range includes a window covering 200-500 nm, both for morin and for the compounds synthesized in Example 1, which guarantees, at least, the absorption of UVB, UVA and UVA-vis rays, since it absorbs above 400 nm.

The tangible improvement with chemical modification for the compounds synthesized in Example 1 is evident by comparing the molar extinction coefficient (ε) values. The results showed that morin oxime exhibited the best ε value, followed by morin semicarbazone. In a further analysis, it can be seen that the *ε*ₘₐₓ of morin oxime is 60% better than that of morin, and *ε*ₘₐₓ of morin semicarbazone is 40% better than that of morin. Based on these results, it can be deduced that one equivalent of either morin oxime or morin semicarbazone is more effective than one of morin in UV-vis absorption, and therefore lower concentrations of these derivatives are necessary to achieve UV-vis absorption values similar to those of morin. Therefore, an improvement can be achieved in the optimization of active ingredients used in the preparation of compositions, the purpose of which is directed towards photoprotection and a photoabsorbent flavonoid is contemplated in said formulation.

### Example 3. Analysis of the antioxidant capacity of morin and the compounds obtained in Example 1

As mentioned above, the aim of these studies is to compare the photoprotective activity of morin, already previously known, with that of two new derivatives obtained from structural modifications of this flavonoid synthesized in Example 1.

First, the antioxidant capacity of the compounds was determined directly using the ABTS technique (2,2-azino-bis-3-benzo-thiazoline-6-sulfonic acid) at different concentrations of the compounds (morin and the compounds of Example 1), using as a reference control a curve of increasing concentrations of Trolox. The results showed that the 2 compounds obtained in Example 1 had an antioxidant activity similar to that of morin and in all cases superior to Trolox (Table 1).

**Table 1. Antioxidant capacity of compounds**

| Sample | EC 50 (*µ*M) | Trolox equivalency (EC50 sample/EC 50 Trolox) |
|---|---|---|
| Trolox | 20.789 ± 0.811 | 1 |
| Morin | 10.750 ± 2.207 | 0.517 |
| Morin oxime | 13.675 ± 0.768 | 0.658 |
| Morin semicarbazone | 12.263 ± 0.922 | 0.590 |

### Example 4. Study of the anti-inflammatory activity of morin and the compounds obtained in Example 1

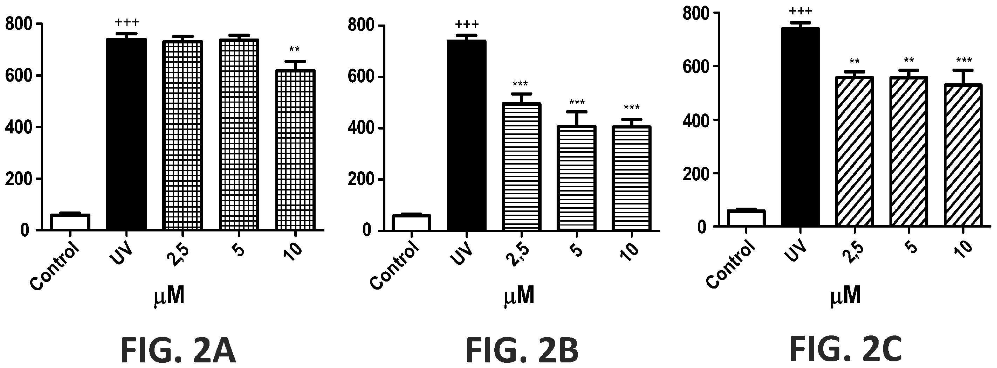

For the evaluation of anti-inflammatory activity (Figure 2), HaCaT keratinocytes were pretreated for 4 h with the compounds morin (A), morin oxime obtained in Example 1 (B) and morin semicarbazone obtained in Example 1 (C) (2.5, 5 and 10 µM). They were then irradiated with UVB radiation at 50 mJ/cm², incubated for 24 h and IL-6 (Interleukin-6) production was assessed by ELISA. Results were expressed as pg/mL and bars represent ± SEM (n=5). The mean was significantly different compared to non-irradiated control cells (+++ p < 0.001; Student's t test). The mean was significantly different compared to irradiated control cells (** p < 0.01, *** p < 0.001; Kruskal-Wallis test followed by Dunn's multiple comparison test). The results showed a potent reduction in IL-6 production after treatment with the compounds of Example 1 at all concentrations tested compared to morin. Thus, it is worth noting that at the highest concentration tested (10 µM), morin oxime reduced IL-6 levels by 45% (Figure 2B) and morin semicarbazone by 28% (Figure 2C), compared to morin, with which a decrease of only 16% was observed (Figure 2A).

### Example 5. Evaluation of the antioxidant activity of morin semicarbazone

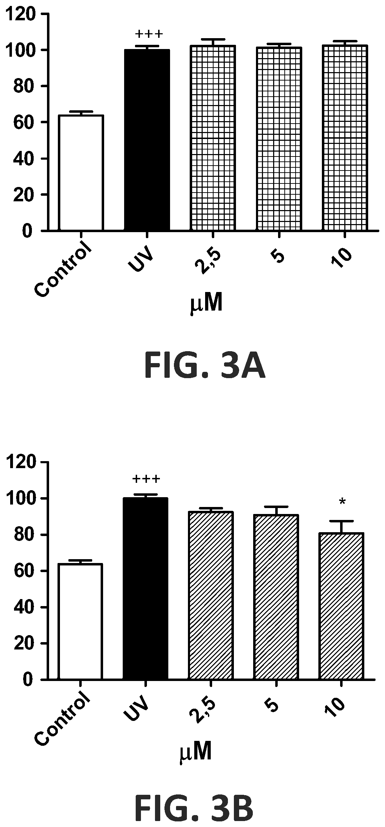

For the evaluation of the antioxidant activity of morin semicarbazone obtained in Example 1, HaCaT cells were pre-treated with morin and semicarbazone at the selected concentrations for 4 h and then irradiated with UVB radiation at 50 mJ/cm². After 45 min, ROS levels were measured using the dichlorodihydrofluorescein-diacetate technique. The results of these studies showed that the morin semicarbazone derivative reduced ROS production by up to 20% at a concentration of 10 µM (Figure 3B), compared to morin (Figure 3A), which failed to reduce ROS levels at any of the concentrations tested. The results were expressed as a percentage with respect to the irradiated cell control and the bars represented mean ± SEM (n=4). The mean was significantly different compared to non-irradiated control cells (+++ p < 0.001; Mann-Whitney U test). The mean was significantly different compared to irradiated control cells (* p < 0.05, ** p < 0.01; Kruskal-Wallis test followed by Dunn's multiple comparison test).

### Example 6. Testing the combination of morin or semicarbazone with fucoxanthin

Once the antioxidant and anti-inflammatory activity was assessed, synergy studies were performed. For this purpose, morin semicarbazone from Example 1 was selected and combined in 1:1 proportions with fucoxanthin, a carotenoid with known photoprotective activity.

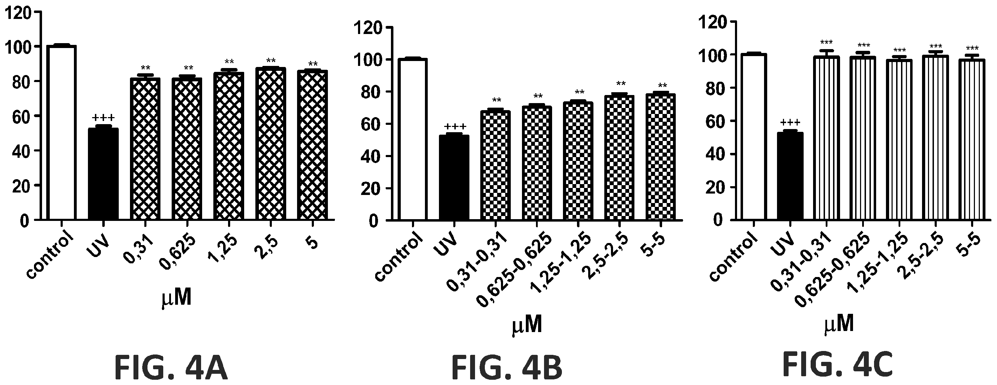

The results of this study showed that the combination of the morin semicarbazone derivative from Example 1 with fucoxanthin (Figure 4C) induced greater protection of cell viability than the carotenoid alone (Figure 4A), managing to recover viability to levels of healthy cells at all concentrations tested. Furthermore, this combination was much more effective than when fucoxanthin was used combined with the reference compound morin, wherein values around 70-80% were obtained (Figure 4B).

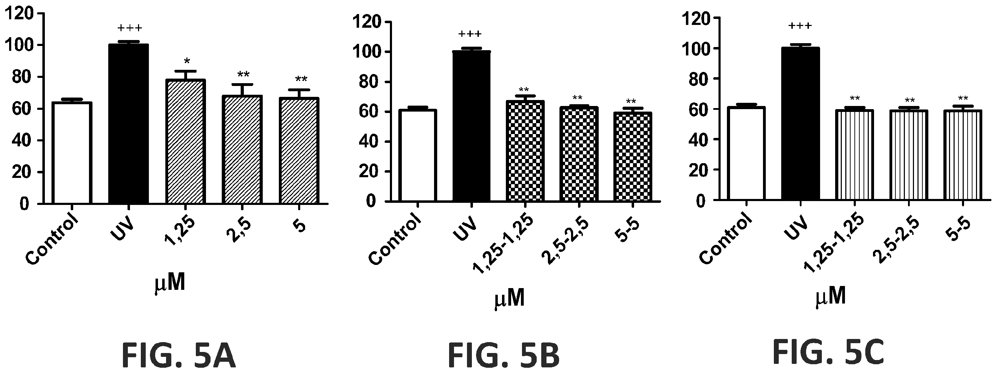

Regarding ROS production, both morin and the morin semicarbazone derivative of Example 1, combined with fucoxanthin in a 1:1 proportion, significantly reduced the levels of this marker at all concentrations tested, reaching levels comparable to those of non-irradiated cells. This decrease was slightly greater with the fucoxanthin+morin semicarbazone mixture at the 1.25-1.25 µM and 2.5-2.5 µM combinations (41% reduction) (Figure 5C) than with fucoxanthin+morin (33% and 37% reduction, respectively) (Figure 5B). Furthermore, it should be noted that the combination of the derivative with fucoxanthin was more effective than when fucoxanthin was used alone, with which reductions of 34% were observed with the highest concentration tested (Figure 5A). The results were expressed as a percentage with respect to the irradiated control cells and the bars represented mean ± SEM (n = 4). The mean was significantly different compared to the non-irradiated control cells (+++ p < 0.001; Mann-Whitney U test). The mean was significantly different compared to the irradiated control cells (* p < 0.05, ** p < 0.01; Kruskal-Wallis test followed by a Dunn's multiple comparison test).

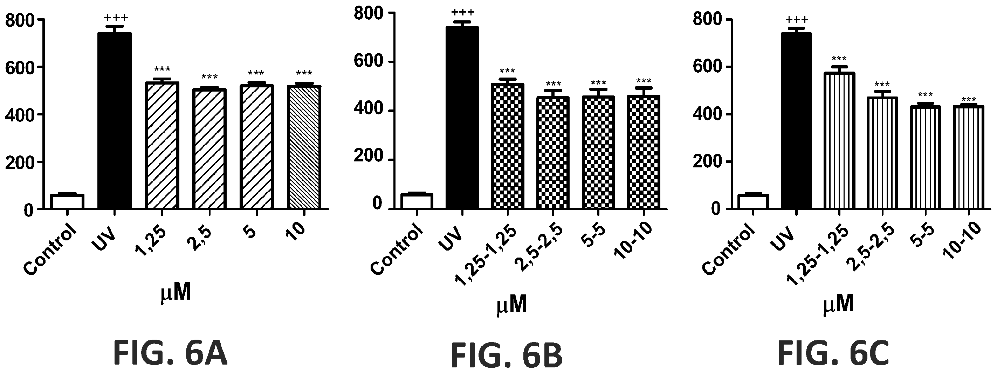

Finally, the combination of morin or the morin semicarbazone derivative from Example 1 with fucoxanthin induced a decrease in IL-6 production at all concentrations tested in a 1:1 proportion. It is worth mentioning that this reduction was more notable with the fucoxanthin+morin semicarbazone mixture at the 5-5 µM and 10-10 µM combinations (42% reduction) (Figure 6C) than with fucoxanthin+morin (38% reduction) (Figure 6B). Furthermore, the combination of the derivative with fucoxanthin was more effective than when the carotenoid was used alone, with which reductions of 30% were observed with the highest concentration tested (Figure 6A). The results were expressed as a percentage with respect to the irradiated control cells and the bars represented mean ± SEM (n = 4). The mean was significantly different compared to the non-irradiated control cells (+++ p < 0.001; Student's t test). The mean was significantly different compared to the irradiated control cells (*** p < 0.001; Kruskal-Wallis test followed by a Dunn's multiple comparison test).

## Claims

1. A compound of formula (I) 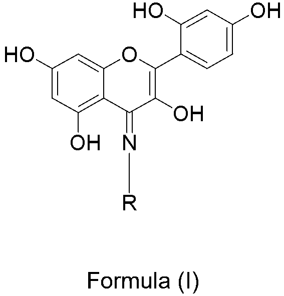
wherein R is selected from: a straight or branched saturated C₁-C₆ alkyl radical, a straight or branched C₂-C₆ alkenyl radical; a straight or branched C₂-C₆ alkynyl radical, a C₃-C₁₂ cycloalkyl radical, a C₃-C₁₂ aryl radical, a -XR¹ radical, a --NHC=XNR²R³ radical, a -NHR⁴ radical, a -NHCOOR⁵ radical, a -NHCOR⁶ radical;
wherein X is selected from O, S;
wherein R¹, R², R³, R⁴, R⁵, and R⁶ may be the same or different and are selected from H, a straight or branched saturated C₁-C₆ alkyl radical, a straight or branched C₂-C₆ alkenyl radical; a straight or branched C₂-C₆ alkynyl radical, a C₃-C₆ cycloalkyl radical, a C₃-C₆ aryl radical.

2. The compound of formula (I) **characterized in that** it is morin oxime, (2-(2,4-dihydroxyphenyl)-3,5,7-trihydroxy-4H-chromen-4-one oxime).

3. The compound of formula (I) **characterized in that** it is the morin semicarbazone, (2-(2-(2,4-dihydroxyphenyl)-3,5,7-trihydroxy-4H-chromen-4-ylidene)hydrazine-1-carboxamide).

4. A method for obtaining compounds of general formula (I) as defined in claim 1 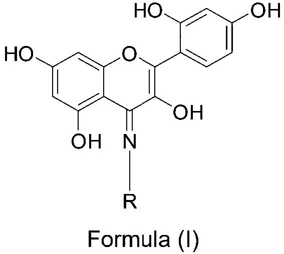 which comprises the steps of:
reacting 2-(2,4-dihydroxyphenyl)-3,5,7-trihydroxy-4H-chromen-4-one, of formula (II) 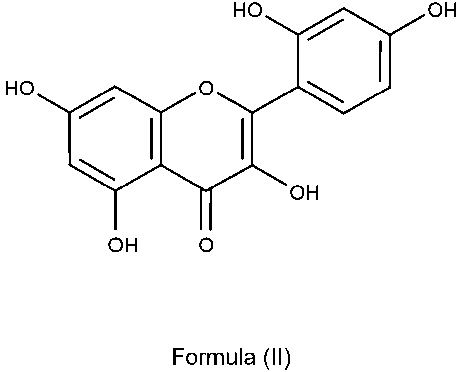
with a nitrogen compound of formula H₂NR, in the presence of an organic solvent; wherein R is selected from: a saturated, straight or branched C₁-C₆ alkyl radical, a linear or branched C₂-C₆ alkenyl radical; a linear or branched C₂-C₆ alkynyl radical, a C₃-C₁₂ cycloalkyl radical, a C₃-C₁₂ aryl radical, a -XR¹ radical, a -NHC=XNR²R³ radical, a -NHR⁴ radical, a -NHCOOR⁵ radical, a -NHCOR⁶ radical;
wherein X is selected from O, S;
wherein R¹, R², R³, R⁴, R⁵, and R⁶ may be the same or different and are selected from H, a straight or branched C₁-C₆ saturated alkyl radical, a linear or branched C₂-C₆ alkenyl radical; a linear or branched C₂-C₆ alkynyl radical, a C₃-C₆ cycloalkyl radical, a C₃-C₆ aryl radical.

5. The method for obtaining the compounds of formula (I) according to claim 4, **characterized in that** the nitrogen compound that reacts with the compound of formula (II) is hydroxylamine hydrochloride or semicarbazide hydrochloride.

6. The method of obtaining according to claim 4 or 5 **characterized in that** the organic solvent is methanol.

7. Use of a product of formula (I) according to claim 1 in the preparation of a cosmetic or dermatological composition to protect a mammal from UV-vis radiation.

8. Compound of formula (I) according to any of claims 1 to 3 for use in medicine or dermocosmetics.

9. Compound of formula (I) according to any of claims 1 to 3 for use in protecting a mammal from UV-vis radiation.

10. Compound of formula (I) according to any of claims 1 to 3 for use in the prevention of skin cancer.

11. Compound of formula (I) according to any of claims 1 to 3 for use in preventing inflammation, oxidative stress and skin aging.

12. Compound of formula (I) according to any of claims 1 to 3 for use as an adjuvant for photoprotectors.

13. Compound of formula (I) according to claim 12 wherein the photoprotector is fucoxanthin.

14. Cosmetic composition or dermatological composition comprising the product of formula (I) according to any of claims 1 to 3.

15. Cosmetic composition or dermatological composition comprising (2-(2-(2,4-dihydroxyphenyl)-3,5,7-trihydroxy-4H-chromen-4-ylidene)hydrazine-1-carboxamide) and fucoxanthin.
